# EUROPEAN PATENT SPECIFICATION

(11) **EP 1 389 645 B1**
(45) Date of publication and mention of the grant of the patent: **16.02.2011**
(21) Application number: 03016647.4
(22) Date of filing: 31.07.2003
(51) Int. Cl.: D06M 23/16, D06M 11/83, D06M 13/00, D06M 15/00

(54) **High protection against uv radiation fabric and process for the manufacture thereof**
Gegen UV-Strahlung geschützes Material und dessen Herstellung
Textile de haute protéction contre les radiations U.V. et prodédé de fabrication

(30) Priority: 02.08.2002 IT BO20020510; 02.08.2002 IT BO20020511
(43) Date of publication of application: 18.02.2004
(73) Proprietor: Massimo Guarducci S.r.l., 59100 Prato (IT)
(72) Inventor: Sarti, Paolo, 59100 Prato (IT); Sarti, Roberto, 59100 Prato (IT); Guarducci, Massimo, 59100 Prato (IT)
(74) Representative: Pipparelli, Claudio

(56) References cited:
- EP-A- 0 825 188
- DE-A- 10 029 284
- DE-A- 10 105 143
- US-A- 6 025 284
- DATABASE WPI Section Ch, Week 199248 Derwent Publications Ltd., London, GB; Class A94, AN 1992-392451 XP002265876 & JP 04 289268 A (KANEBO LTD), 14 October 1992 (1992-10-14)

## Description

The present invention is pertaining to the technical field relevant to the fabric manufacture.

Particularly the invention refers to a novel fabric, obtained by means of knitting machines or common looms, capable to grant a very high protection degree from the UV radiations, as well as to the process for the manufacture of the same.

It is known that, in order to improve the skin protection against the UV radiations, some fabrics aimed to make clothing items are built with peculiar yarns or mixtures thereof, or they undergo treatments with products increasing their adsorption or reflection factor of the very UV radiations.

US patent n. 5.503.917 discloses a fabric having a high UV protection degree made by a nylon yarn of at least 40x40 deniers and a density of at least 80x50 filaments. The fabric is sandblasted on a side and air jet washed.

Another fabric having a high UV protection is described in US patent n. 6.025.284. Such a fabric is made by polyester yarn and is treated with UV absorbing products, such as chlorobenzotriazoles. Polyester is furtherly treated to stand wrinkled.

US patent n. 6.034.003 disclosed a method for obtaining an UV high absorption fabric. The fabric is immersed into a solution of antraquinone and NaOH, and then heated. The fabric containing solution is then added by a reducing agent, and thereafter the fabric is rinsed with hydrogen peroxide. The white fabrics are then subjected to a conventional bleaching, while the coloured fabrics are added by a further UV absorbing compound containing melanine.

The abovementioned fabrics give a good protection against UV radiations, even if they are not particularly comfortable, which let the same be not suitable to make children clothing items, sports clothing items, or items aimed to a direct contact with the skin, above all for long time periods.

One purpose of the present invention is a process for the manufacture of a dual side fabric having a high protection factor of the UV radiations, being in the same so comfortable to be employed in the child dress, in the sport dress, as well as for clothing items aimed to a prolonged contact with skin, and in any case for all applications in which a peculiar outlining is given to the skin safety of the present fabric user, who may have a direct contact therewith.

The above purposes are fully achieved according to the claim content.

The invention characteristics, as from the claim definitions, are outlined by the following detailed specification.

According to the inventive process, the high UV radiation protection fabric is made by two different yarns as a double side fabric, according to known wearing techniques. The fabric obtained thereby shows an external side wherein a first yarn does appear, and an inner side wherein a second yarn does appear. In such a way, through a suitable selection of the composition and the structure of the two yarns, it is possible to furtherly improve the fabric comfort characteristics.

In the above embodiment, the fabric external side is constituted by a textile skeleton made by a yarn for instance a polyester yarn, if necessary suitably treated. The fabric may be made by means of a knitting machine or of a conventional loom.

The yarn undergoes a particular treatment : consisting in adding the yarn a predetermined amount of a product reflecting UV radiations of the ceramic or metallic kind. These products are well known in the textile field, as well as the processes for putting the same into the yarn skeleton. For istance, it is possible to employ the yam commercially known as "Polyester Yam UV Shield Ultra Dull Ceramic" manufactured by "Syntraco" (R) company. Such yam brings, enclosed inside its skeleton, a ceramic reflecting product. Some other equivalent products can be advantageously used, for instance yarns the reflecting products have been applied on the surface of. These products grant the yam high reflecting power towards radiations in the UV range, either UVA or UVB. To gain a good reflecting property, the above described yarns get percentages of reflecting products generally ranging from 1% to 5% by volume.

In order to make the fabric internal side, in any case use is made of an artificial, synthetic or natural yam having high comfort and wearability characteristics. This yarn does not ever undergo the abovementioned reflecting product englobing treatment, which let the skin contact be more comfortable.

The fabric produced by means of the abovementioned yarns then undergoes operations usually performed to obtain a finished product. Such operation comprise the fabric painting and finishing.

According to the invention, during the painting step, the painting bath is added by a product suitable to absorb the UV radiations, which is soaking the fabric together with the dye product and is sharing all over the very fabric quite evenly. These UV absorbers are well known to the skill people. Also known is the resistance thereof against the fabric repeated washing. With reference to the present application, a peculiar suitable use is made of an oxalanilide base powdery product, commercially known as "Tinofast PES" (R) , produced by "Ciba Specialty Chemicals" (R), that is dissolved in the painting bath. The concentration of this products in the painting bath, according to the adsorbing power to be gathered and to bearable product manufacturing cost, ranges from 0.1% to 20% by weight. Preferred concentrations are used in the 1% ÷ 4% b.w.range. The permanence time of the fabric in the painting bath obtained thereaccording is ruled in such a way that the very product and the dye are adsorbed in optimal proportions.

The fabric is then dried by any conventional process, and thereafter subjected to the usual finishing operations, to let the same assume the wanted look.

During the finishing step, the fabric is furtherly impregnated with a skin shield cream, having a high absorbing factor towards UV radiation of the UVA and UVB kind. This cream is aimed to perform a dual function, i.e. the one furtherly adsorbing the ultraviolet radiation striking the clothing item made by the present fabric, and the other one encreasing the comfort properties thereof, mainly the skin protection of the user wearing that item.

The shielding cream employed according to the inventive process must have peculiar characteristics. First it has to be waterproof; then it must have a very high fixing degree onto fabrics.

Particularly employable is the product commercially known as "O Sole Mio" of the "Bambini Soul" line produced by "Massimo Guarducci S.r.l." company.

This cream formulation contains a peculiar component combination, made through a technique commercially known as "Tecnoguard".

The application of the shielding cream onto the fabric through the impregnation process grant obtaining an even and wash steady distribution. However, the same cream may be advantageously applied also through other known techniques, may be also in function of particular production needs. For istance, the application may be carried out through exhaustion, atomization, spaying, surface stratification by spreading or by scraping. Through some of the above application techniques, the shielding cream may he applied only onto one side of the fabric, or onto both of them.

In order to improve the shielding cream adsorption, the very fabric may be also subjected to previous treatments, warm or cold with hard, softened or osmosis water.

It is anyhow possible, to apply only the shielding cream during finishing, and not the UV absorber, when a lower adsorbing factor is enough.

The abovereferred novel fabric, as well as the process for the manufacture thereof, allow to obtain some advantages, with respect to the known fabrics, either with reference to the total fraction of forbidden UVA and UVB radiations, or with reference to the user comfort and the relevant skin protection.

## Claims

1. Process for the manufacture of a dual side fabric having a high protection power against UV radiations and a high wearability **characterized in that** it comprises the following steps: processing, by means of a loom or a knitting machine, a first yam, which contains at least one UV reflecting product of the metallic and ceramic kind, aimed to build the external side of said fabric, and a second yarn, which does not ever contain said UV reflecting product and has a high wearability, aimed to build the inner side of said fabric, to obtain a dual side fabric; treating said dual side fabric during the finishing step with a prefixed amount of a skin shielding cream, having an UV radiation high absorbtion power and a washing high resistance.

2. Process according to claim 1 **characterized in that**, during a painting step of said fabric before the finishing step, the painting bath is added by at least a washing proof UV absorber aimed to soak said fabric.

3. Process according to claim 2 **characterized in that** said UV absorber is contained in said bath in the range from 0,1% to 20% by weight.

4. Process to claim 1, **characterized in that**, said reflecting product is enclosed inside the inner skeleton of said first yarn.

5. Process according to claim 1, **characterized in that**, said reflecting product is applied to the said surface of said first yarn.

6. Process according to claim 1, **characterized in that** the external side of said fabric is furtherly soaked with an UV absorbing washing proof product.

7. Process according to claim 1, **characterized in that** said anti-UV shielding cream is made liquid proof, particularly water proof, and underwent a treatment to improve its fabric fixing.

8. Process according to claim 1, **characterized in that** said anti-UV shielding cream is applied to said fabric by impregnation.

9. Process according to claim 1, wherein the fabric external side is constituted by a textile skeleton made by a yam of polyester.

10. Process according to claim 2, wherein the painting bath is added by an oxalanilide base powdery product.

## Patentansprüche

1. Verfahren für die Herstellung eines zweiseitigen Stoffes, der eine hohe UV-Strahlenschutzleistung und gute Tragbarkeit aufweist, **dadurch gekennzeichnet, dass** es die folgenden Schritte umfasst: Verarbeiten mit Hilfe eines Webstuhles oder einer Strickmaschine eines ersten Garns, das wenigstens ein UV-reflektierendes Erzeugnis einer metallischen und keramischen Art enthält und dazu bestimmt ist, die Außenseite des Stoffes zu bilden, und eines zweiten Garns, das nicht das UF-reflektierende Erzeugnis enthält und eine gute Tragbarkeit aufweist und dazu bestimmt ist, die Innenseite des Stoffes zu bilden, um einen zweiseitigen Stoff zu erzeugen, und Behandeln des zweiseitigen Stoffes während des Endbearbeitungsschrittes mit einer zuvor festgelegten Menge einer Hautabschirmcreme, die eine hohe UV-Strahlungsabsorptionsleistung und eine hohe Waschbeständigkeit hat.

2. Verfahren nach Anspruch 1, **dadurch gekennzeichnet, dass** während eines Färbungsschrittes vor dem Endbearbeitungsschritt dem Färbebad wenigstens ein waschfester UV-Absorber hinzugefügt wird, der dazu bestimmt ist, den Stoff zu tränken.

3. Verfahren nach Anspruch 2, **dadurch gekennzeichnet, dass** der UV-Absorber in dem Bad mit einem Anteil von 0,1 bis 20 Gew-% enthalten ist.

4. Verfahren nach Anspruch 1, **dadurch gekennzeichnet, dass** das reflektierende Erzeugnis in dem inneren Aufbau des ersten Garns enthalten ist.

5. Verfahren nach Anspruch 1, **dadurch gekennzeichnet, dass** das reflektierende Erzeugnis auf die Oberfläche des ersten Garns aufgebracht wird.

6. Verfahren nach Anspruch 1, **dadurch gekennzeichnet, dass** die Außenseite des Stoffes weiterhin mit einem UV-absorbierenden, waschfesten Erzeugnis getränkt wird.

7. Verfahren nach Anspruch 1, **dadurch gekennzeichnet, dass** die Anti-UV-Abschirmcreme flüssigkeitsfest, insbesondere wasserfest, gemacht wird und einer Behandlung unterzogen wurde, um deren Fixierung in dem Stoff zu verbessern.

8. Verfahren nach Anspruch 1, **dadurch gekennzeichnet, dass** die Anit-UV-Abschirmcreme auf den Stoff durch Imprägnieren aufgebracht wird.

9. Verfahren nach Anspruch 1, bei dem die Stoffaußenseite aus einem Textilaufbau besteht, der aus einem Garn aus Polyester gefertigt ist.

10. Verfahren nach Anspruch 2, bei dem dem Färbebad ein Pulvererzeugnis auf Oxalanilidbasis hinzugefügt wird.

## Revendications

1. Procédé de fabrication d'un tissu double face présentant une résistance élevée contre les rayonnements UV et une portabilité élevée, **caractérisé en ce qu'**il comprend les étapes suivantes :
• ennoblissement, au moyen d'une machine à tisser ou à tricoter, d'un premier fil, qui contient au moins un produit de type métallique ou céramique réfléchissant les rayonnements UV, destiné à la fabrication de la face externe dudit tissu, et d'un second fil qui ne contient aucun produit réfléchissant des rayonnements UV et qui présente une portabilité élevée, destiné à la fabrication de la face interne dudit tissu, pour obtenir un tissu double face ;
• traitement dudit tissu double face au cours d'une étape de finissage avec une crème de protection pour la peau, présentant un pouvoir élevé d'absorption des rayonnements UV et une résistance élevée au lavage.

2. Procédé selon la revendication 1, **caractérisé en ce qu'**au cours d'une étape de teinture dudit tissu, avant l'étape de finissage, un absorbant des rayonnements UV résistant au lavage est ajouté au bain de teinture dans lequel le tissu est trempé.

3. Procédé selon la revendication 2, **caractérisé en ce que** ledit absorbant des rayonnements UV est contenu dans ledit bain selon une gamme comprise entre 0,1 et 20% en poids.

4. Procédé selon la revendication 1, **caractérisé en ce que** ledit produit réfléchissant est renfermé dans l'âme dudit premier fil.

5. Procédé selon la revendication 1, **caractérisé en ce que** ledit produit réfléchissant est appliqué sur la surface dudit premier fil.

6. Procédé selon la revendication 1, **caractérisé en ce que** la face externe dudit tissu est en outre trempée dans un produit d'absorption des rayonnements UV résistant au lavage.

7. Procédé selon la revendication 1, **caractérisé en ce que** ladite crème de protection anti-UV est produite de manière à être imperméable aux liquides, particulièrement imperméable à l'eau, et **en ce qu'**elle est soumise à un traitement pour améliorer son adhérence au tissu.

8. Procédé selon la revendication 1, **caractérisé en ce que** ladite crème de protection anti-UV est appliquée sur ledit tissu par imprégnation.

9. Procédé selon la revendication 1, dans lequel la face externe du tissu est constituée d'une trame tissu formée d'un fil de polyester.

10. Procédé selon la revendication 2, dans lequel un produit en poudre à base d'oxalanilide est ajouté au bain de teinture.
